# EUROPEAN PATENT SPECIFICATION

(11) **EP 2 459 235 B1**
(45) Date of publication and mention of the grant of the patent: **23.04.2014**
(21) Application number: 10740399.0
(22) Date of filing: 21.07.2010
(51) Int. Cl.: A61K 49/10, A61K 49/18, B82Y 5/00

(54) **PERFLUORO-T-BUTYL CYCLOHEXANE FOR USE IN IMAGING**
PERFLUORTERTBUTYLCYCLOHEXAN ZUR BILDGEBUNG
PERFLUORO-T-BUTYL CYCLOHEXANE POUR L'IMAGERIE

(30) Priority: 31.07.2009 EP 09166955
(43) Date of publication of application: 06.06.2012
(73) Proprietor: Koninklijke Philips N.V., 5656 AE Eindhoven (NL)
(72) Inventor: YILDIRIM, Muhammed, NL-5656 AE Eindhoven (NL); GRUELL, Holger, NL-5656 AE Eindhoven (NL); LAMERICHS, Rudolf, Mathias, Johannes, Nicolaas, NL-5656 AE Eindhoven (NL)
(74) Representative: van Velzen, Maaike Mathilde
(86) International application number: PCT/IB2010/053321
(87) International publication number: WO 2011/013038

(56) References cited:
- WO-A2-2009/154425
- US-A1- 2003 185 760
- LIN W H ET AL: "The synthesis of highly fluorinated alkylcyclohexanes for use as oxygen carriers and the <19>F and <13>C NMR spectra of alkylcyclohexanes", JOURNAL OF FLUORINE CHEMISTRY, ELSEVIER, NL, vol. 50, no. 3, 1 December 1990 (1990-12-01), pages 345-358, XP026619658, ISSN: 0022-1139, DOI: DOI:10.1016/S0022-1139(00)85000-9 [retrieved on 1990-12-01]
- DE LANGE F. ET AL.: "Perfluorocarbon Administration During Cardiopulmonary Bypass in Rats: An Inflammatory Link to Adverse Outcome?", ANESTHESIA AND ANALGESIA, vol. 106, no. 1, 1 January 2008 (2008-01-01), pages 24-31, XP008133214,

## Description

### FIELD OF THE INVENTION

The invention pertains to the use of perfluoro compounds and emulsions thereof in imaging, e.g. molecular imaging. Particularly, the invention pertains to the use of said compounds and nanoparticle emulsions thereof in ¹⁹F MRI and in multi-modality imaging.

### BACKGROUND OF THE INVENTION

In Molecular Imaging (MI) purposes, specific disease markers in the body are visualized by imaging. One of the key elements in this MI approach is, that the markers are targeted by a contrast agent that can be visible on a medical imaging modality, e.g. magnetic resonance imaging (MRI), single photon emission computed tomography (SPECT), positron emission tomography (PET), computed tomography (CT). For example, MRI is one of the major diagnostic imaging techniques in medicine. MRI generates detailed images of soft tissues. For this purpose, the magnetic properties of the hydrogen atoms of water and fat are used.

Morawski et al., Magn. Reson. Med. 52, 1255 (2004), describe the quantitative molecular imaging of fibrin, using a non-aqueous ¹⁹F contrast agent, viz. ligand-targeted perfluoro nanoparticles. Morawski describes the use of ¹H MRI as well as ¹⁹F MRI.

Magnetic Resonance Imaging based on ¹⁹F instead of ¹H opens up new diagnostic possibilities. Since ¹⁹F does not naturally occur in the body, ¹⁹F MRI will necessarily be based on the use of added ¹⁹F contrast agents. This eliminates the need for a so-called, pre-contrast scan. Generally this makes the diagnostic imaging procedure significantly easier. Furthermore, as only signals from these agents are detected, ¹⁹F MRI has a high specificity.

In order to have sufficient sensitivity of the ¹⁹F, the contrast agent will generally comprise an emulsion of ¹⁹F compound. The size of the particles will be typically between 200-1000 nm, large enough to deliver sufficient ¹⁹F nuclei to the site-of-interest, allowing for detection of small numbers of markers.

An advantage of an emulsion of perfluoro nanoparticles is that they can also carry other molecules to the site-of-interest. These molecules can be 'attached' to the outside. E.g., gadolinium chelates can be anchored in the hydrophobic outer layer, making the agents also visible on T1 weighted proton MRI. Similar, radioactive labels that are detectable by SPECT can be added as well. These multi-modality agents can be detected by both MRI and SPECT. Also the delivery of therapeutic agents can be possible.

In order to be suitable for the aforementioned use, a perfluoro-compound needs to be non-toxic to humans, and be capable of forming stable emulsions with the right particle size. These two requirements often conflict with yet another desire, viz. avoiding too long a retention time of the compound in the body.

Although many non-toxic ¹⁹F based agents are available, these compounds frequently have a much too long retention time in the body (Jean G. Riess, Chem. Rev. 2001, 101, 2797-2919). When the emulsion particles comprising such perfluoro compounds fall apart, as is the intention, the per-F compounds, which are extremely hydrophobic, tend to settle into fat and are retained there for an undesirably long time (weeks, months and even longer).

A reference to the use of perfluorocarbons in ¹⁹F MRI, is WO 91/12824. Whilst this document presents a broad teaching, in the rapidly developing field of ¹⁹F contrast agents it is an ongoing desire to find compounds that have a relatively large number of detectable ¹⁹F, that are capable of forming relatively stable emulsions, and that exhibit a relatively short retention time in the body, preferably less than a week.

US2003/185760 discloses contrast agents for magnetic resonance imaging comprising nanoparticles provided with a core containing several perfluoroalkanes.

### SUMMARY OF THE INVENTION

In order to better address the foregoing desire, the invention, in one aspect, presents the use of perfluoro-t-butyl cyclohexane as a contrast agent for molecular imaging, and particularly as a contrast agent for ¹⁹F MRI and in mixed imaging modalities including, T1 weighted 1H MR and nuclear medicine. More in particular, in another aspect, the invention provides the use of emulsions comprising perfluoro-t-butyl cyclohexane as a contrast agent in molecular imaging. In yet another aspect, the invention provides imaging methods wherein use is made of perfluoro-t-butyl cyclohexane, and emulsions comprising perfluoro-t-butyl cyclohexane. Also, in still another aspect, the invention presents perfluoro-t-butyl cyclohexane, and particularly emulsions comprising perfluoro-t-butyl cyclohexane, for use in imaging methods practiced on the human or animal body.

### BRIEF DESCRIPTION OF THE DRAWING

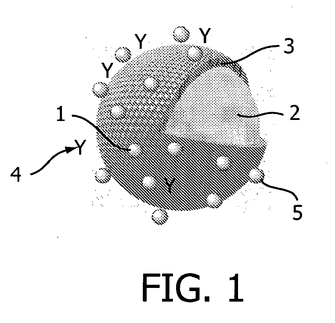

Fig. 1 depicts a nanoparticle (1) comprising a core (2) of perfluoro tertiary-butyl cyclohexane. This core is surrounded by a shell (3), which may be, for example, a phospholipid shell. This lipid shell may be provided with a ligand for targeted binding (4), e.g. antibodies. A gadolinium chelate (5) or a radioactive SPECT tracer may also be provided by the lipid shell.

### DETAILED DESCRIPTION OF EMBODIMENTS

The present invention is based on the insight that perfluoro-t-butyl cyclohexane (PtBC) has an excellent set of combined properties for use as a contrast agent in imaging, particularly molecular imaging. This set of properties, in combination, comprises non-toxicity to animals and humans;
a high number of fluorine atoms in a relatively small molecule;
a short time of retention in the animal or human body;
ability to make stable emulsions

The compound thus selected according to the invention satisfies the following molecular formula:

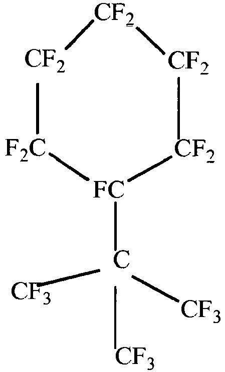

With 20 fluorine atoms it is an agent capable of providing a suitable contrast in ¹⁹F MRI and therewith presents a favourable alternative to a much suggested compound, perfluoro octyl bromide.

Besides being suitable for use in ¹⁹F MRI, the agent of the invention can also be used in ¹H MRI in a method as disclosed in WO 95/33494.

The compound PtBC itself is known, and obtaining it e.g. by chemical synthesis does not present any particular problem to the skilled person.

The contrast agent of the invention is preferably in the form of an emulsion of PtBC. Emulsion of perfluorinated liquids, in the case of a variety of other perfluorinated hydrocarbons, are known. Typically, the PtBC together with a suitable emulsifier is present as a stable emulsion in a continuous aqueous phase. The hydrophobic perfluoro carbon thereby is surrounded by a surfactant layer, i.e. providing a stabilized (nano)particle having a surfactant monolayer that is hydrophilic to the outside and hydrophobic to the inside.

Thus the contrast agent of the invention in effect comprises nanoparticles of the perfluorocarbon. Preferred nanoparticle contrast agents are based on a perfluorocarbon core and a (phospho)lipid layer encapsulating the core (i.e. a shell). The layer has two functions; to stabilize the particle and to allow for interaction with ligands used for targeted binding, as well as for interaction with agents used for multi-modality imaging, such as Gd chelates for ¹H MRI and/or radionuclides for SPECT or PET. The size of these particles is generally in the nanometer range (50-1500 nm, preferably 200-1000 nm). One could also use particles with a shell made from polymers, which are filled with a perfluoro-compound. E.g. a preferred type of such particles as can be used in the invention, and a method of making them, is described in WO 2006/046200 (there used for ultrasound imaging).

To the outer hull of any of these particles the ligands for specific targeted binding, as well as agents for multi-modality imaging, can be attached. This can be done in various known manners, *inter alia* through covalent bonding (e.g. to a polymeric outer hull), by having hydrophobic tails penetrate into the surface of the particle (e.g. in the case of a phosholipid surface), or by using e.g. biotine - streptavidine linking.

The use of the nanoparticles according to the invention is not limited to any particular target. On the other hand, the improved ¹⁹F MRI method which results from using the complexes, and resulting nanoparticles, of the invention, are believed to enable improvements in relation to such disorders as require improvement in the detection thereof. Thus, the invention is believed to be particularly useful in the detection of tumors such as in colorectal cancer, e.g. based on CEA (carcinoembryonic antigen) or MUC1 as biomarkers, and of cardiovascular disorders such as lead to the presence of vulnerable plaques, for which e.g. fibrine is a biomarker. Also upregulated v₃ integrins in the vascular system can be detected, this can be indication of both arteriosclerosis as well as tumors.

In a preferred embodiment, the PtBC nanoparticles of the present invention are also used as a PET or SPECT detectable agent. In that event, radioactive nuclei are anchored in the lipid layer surrounding the perfluoro carbon. Suitable radioactive nuclei include, but are not limited to, ¹¹C, ¹⁸F, ⁶⁷Ga, ^{99m}Tc, ¹¹¹In, ²⁰¹T1, and ¹²³I.

In another preferred embodiment, the PtBC nanoparticles of the present invention are also used as a contrast agent in T1 weighted ¹H MRI. In that event, suitable chelates of paramagnetic metals are included in the lipid layer, notably gadolinium chelates such as Gd[DOTA] or Gd[DTPA] or Gd[DO3A] complexes. The effect on the relaxivity of the emulsion can be found in: "Winter PM, Caruthers SD, Yu X, Song S-K, Fuhrhop RW, Scott MJ, Chen J, Miller B, Bulte JWM, Gaffney PJ, Wickline SA, Lanza GM. Improved molecular imaging contrast agent for early detection of unstable atherosclerotic plaques. Magn Reson Med 2003;50:411-416.'' It is preferred that the size of the particles is chosen so as to allow placing as many chelates as possible on the surface, as this will enhance T1 relaxation.

An overview on MRI contrast agents can be found in "Contrast Agents I, Magnetic Resonance Imaging", 221 Topics in Current Chemistry, Volume Editor: W.Krause, Springer 2002 and in "Textbook of Contrast Media", Edited by P. Dawson, D. O. Gosgrove, R. G. Grainger, ISIS Medical Media Ltd. Oxford 1999, Section II MR contrast agents, page 251-427.

In another preferred embodiment, the nanoparticles of PtBC are presented in a form that allows detection in ¹⁹F MRI with an improved signal-to-noise ratio, by providing T1 reduction as a result of the presence of a paramagnetic complex comprising at least one paramagnetic metal having at least one coordination site complexed with a fluorophilic ligand. In another preferred embodiment, also capable of leading to a T1 reduction in ¹⁹F, a paramagnetic metal is present on the outside. The paramagnetic metal can be any metal having paramagnetic properties. Paramagnetic metals are known to the skilled person, and do not require elucidation here. E.g., early and late transition metals, explicitly including chromium, manganese, iron, as well as lanthanides, such as gadolinium, europium, dysprosium, holmium, erbium, thulium, ytterbium, and in general ferromagnetic metals above their Curie temperature. The term "metal" denotes neutral metal ions or positively or negatively charged metal ions, or clusters or alloys comprising any combination of those. Many of these metals shorten both T1 and T2. Preferred are those paramagnetic metals which result in the greatest difference of T1 reduction over T2 reduction, of which gadolinium is the most preferred example. A reference on this method of achieving T1 reduction ¹⁹F MRI is WO 2008/132666.

It is further preferred that the agent of the invention, in addition to the ¹⁹F MRI detectable PtBC, comprises a combination of at least two of the foregoing possible attachments to the shell, e.g. gadolinium as well as a ligand for targeted binding, and in addition also a PET or SPECT detectable label can be added if multi-modality imaging is desired.

By the judicious choice of PtBC as a perfluoro compound for making a biocompatible, ¹⁹F MRI detectable emulsion, the present invention provides the advantage to allow for the spectral identification of multiple targets.

For example, a PtBC based agent can be constructed that targets a specific oncology marker, i.e. marker A. Using, PFOB which is also biocompatible, a second agent can be constructed which targets marker B. Since PtBC and PFOB have different NMR spectra they can be distinguished. Now it is possible in one imaging session to determine the ratios of A and B. This can be important, since the ratio can distinguish more clearly between a healthy and a diseased state. Reference is made to WO 2008/114194 which discloses the use of spin species having two different chemical shift spectra. By virtue of the invention, a second per-fluoro compound has now become available that is biocompatible, and therewith can be used together with PFOB so as to provide a unique combination of two biocompatible per-fluoro compounds having different chemical shift spectra.

The agent of the invention can be administered to an animal or human subject in any manner available for the administration of nanoparticle emulsions, including but not limited to intravenous administration, oral administration, rectal administration. Preferably, the agent is injected intravenously or is introduced into the gastro-intestinal tract.

In this respect the invention also pertains to an imaging method comprising the steps of administering, to a human being or an animal, a ¹⁹F MRI contrast agent comprising perfluoro-t-butyl cyclohexane, and subjecting the human being or the animal to scanning by ¹⁹F molecular resonance imaging. MRI scanning procedures, including ¹⁹F MRI, are well-known in the field and do not require elucidation here. The same holds for the MRI equipment suitable for use in such a method.

In order to have a more complete benefit of the foregoing imaging method, it is preferred that the contrast agent used therein is in the form of nanoparticles comprising, in addition to the perfluoro-t-butyl cyclohexane, one or more functionalities in the shell. Reference is made to the description above, from which it will be clear that preferred functionalities include ligands for targeted binding, paramagnetic agents, and radioactive labels. Depending on the set of functionalities, the imaging method of the invention comprises one or more additional steps.

Thus, in one preference, the imaging method of the invention is a targeted imaging method in which use is made of a contrast agent in the form of nanoparticles comprising perfluoro-t-butyl cyclohexane and a ligand for targeted binding, the method comprising the steps of administering the nanoparticles to an animal or a human being, allowing the administered nanoparticles to circulate long enough (typically 6-48 hours, preferably 12-24 hours) to be able to bind to the intended target sites, and subjecting the animal or the human being to scanning by ¹⁹F molecular resonance imaging.

The foregoing method is applicable to any available biological targeting. Specific combinations of targeting moieties, such as antibodies, and targets to be locally imaged are known in the art, and are under constant development in the sense that, as time progresses, new targets will be discovered, and related targeting moieties developed. It will be clear to the person skilled in the art that the contrast agents used in the imaging method of the present invention need not be limited to any specific targeting moiety.

In another preference, the imaging method of the invention is a combined imaging method in which, in addition to ¹⁹F molecular resonance imaging, at least one other imaging method is applied.

This can be ¹H MRI, in which case use is made of a contrast agent in the form ofnanoparticles comprising perfluoro-t-butyl cyclohexane and a contrast agent for ¹H MRI, such as a gadolinium complex as mentioned above. The imaging method of the invention then comprises the steps of administering, to an animal or human being, a combined ¹⁹F and ¹H MRI contrast agent comprising perfluoro-t-butyl cyclohexane and a paramagnetic chemical shift agent, and subjecting the animal or human being to scanning by ¹⁹F and ¹H molecular resonance imaging.

In a particularly preferred embodiment, the other imaging method is SPECT. In this case use is made of a contrast agent in the form of nanoparticles comprising perfluoro-t-butyl cyclohexane and a radio-active label, as described above. The imaging method of the invention then comprises the steps of administering, to an animal or human being, a combined ¹⁹F and radio-imaging contrast agent, comprising perfluoro-t-butyl cyclohexane and a radiolabel, and subjecting the animal or human being to scanning by ¹⁹F resonance imaging and single photon emission computed tomography.

This method too can further be combined with ¹H MRI on the basis of the presence of paramagnetic chemical shift agents, such as Gd chelates on the particle surface. Thus, by way of most preferred embodiment, the imaging method of the invention presents a combination of targeted imaging using combined imaging techniques. The method the involves the administration of a contrast agent in the form of nanoparticles comprising perfluoro-t-butyl cyclohexane, a ligand for targeted binding, and a further contrast agent (particularly an ¹H MRI contrast agent or a radiolabel). The method comprises the respective combined steps.

As will be apparent from the foregoing, the imaging methods described can be applied on the human or animal body. In this respect, the invention also pertains to perfluoro-t-butyl cyclohexane for use in any of the foregoing ¹⁹F MRI methods, targeted imaging methods, and combined imaging methods.

## Claims

1. The use of perfluoro-t-butyl cyclohexane as a contrast agent for imaging, preferably molecular imaging.

2. A use according to claim 1, wherein the imaging comprises ¹⁹F Magnetic Resonance Imaging.

3. A use according to claim 1 or 2, wherein the contrast agent comprises an aqueous emulsion of the perfluoro tertiary-butyl cyclohexane.

4. A use according to claim 3, wherein the emulsion comprises nanoparticles having a core comprising the perfluoro tertiary-butyl cyclohexane surrounded by a shell of an emulsifying agent, such as a phospholipid, the shell comprising a ligand for targeted binding.

5. A use according to claim 3 or 4, wherein the emulsion comprises nanoparticles having a core comprising the perfluoro tertiary-butyl cyclohexane surrounded by a shell of an emulsifying agent, such as a phospholipid, the shell comprising an imaging contrast agent selected from the group consisting of paramagnetic agents, radiolabels, and mixtures thereof.

6. A contrast agent for use in a diagnostic imaging method practised on the human or animal body comprising ¹⁹F MRI, the agent comprising an aqueous emulsion of nanoparticles having a core comprising perfluoro tertiary-butyl cyclohexane surrounded by a shell of an emulsifying agent, such as a phospholipid, the shell comprising at least one further imaging functionality, selected from the group consisting of ligand for targeted binding, ¹H MRI contrast agents, radioactive labels, and mixtures thereof.

7. Perfluoro tertiary-butyl cyclohexane for use as a ¹⁹F molecular resonance imaging contrast agent in a diagnostic imaging method practised on the human or animal body.

8. Perfluoro tertiary-butyl cyclohexane for use in a diagnostic method practised on the human or animal body in accordance with claim 7, wherein the contrast agent comprises an aqueous emulsion of the perfluoro tertiary-butyl cyclohexane,

9. Perfluoro tertiary-butyl cyclohexane for use in a diagnostic method practised on the human or animal body in accordance with claim 8, wherein the emulsion comprises nanoparticles having a core comprising the perfluoro tertiary-butyl cyclohexane surrounded by a shell of an emulsifying agent, such as a phospholipid, the shell comprising a ligand for targeted binding.

10. Perfluoro tertiary-butyl cyclohexane for use in a diagnostic method practised on the human or animal body in accordance with claim 8 or 9, wherein the emulsion comprises nanoparticles having a core comprising the perfluoro tertiary-butyl cyclohexane surrounded by a shell of an emulsifying agent, such as a phospholipid, the shell comprising an imaging contrast agent selected from the group consisting of paramagnetic agents, radiolabels, and mixtures thereof.

11. Perfluoro tertiary-butyl cyclohexane for use in a diagnostic method practised on the human or animal body in accordance with any of the claims 7-10, wherein the contrast agent is injected intravenously or is introduced into the gastro-intestinal tract.

12. Perfluoro tertiary-butyl cyclohexane for use in a diagnostic method practised on the human or animal body in accordance with any of the claims 7-11, wherein a second ¹⁹F MRI contrast agent, preferably per-fluoro octyl bromide, is applied in addition to the perfluoro tertiary-butyl cyclohexane contrast agent.

13. Perfluoro tertiary-butyl cyclohexane for use in a diagnostic method practised on the human or animal body according to claim 12, wherein the perfluoro tertiary-butyl cyclohexane contrast agent is provided with one or more ligands for a first target, such as a first oncology marker, and the second ¹⁹F MRI contrast agent is provided with one or more ligands for a second target, such as a second oncology marker different from the first.

## Patentansprüche

1. Verwendung von Perfluor-tert-Butylcyclohexan als Kontrastmittel für die Bildgebung, vorzugsweise die molekulare Bildgebung.

2. Verwendung nach Anspruch 1, wobei die Bildgebung 19F-Magnetresonanz-Bildgebung umfasst.

3. Verwendung nach Anspruch 1 oder 2, wobei das Kontrastmittel eine wässrige Emulsion des Perfluor-tert-Butylcyclohexan umfasst.

4. Verwendung nach Anspruch 3, wobei die Emulsion Nanopartikel mit einem Kern umfasst, der das Perfluor-tert-Butylcyclohexan umgeben von einer Hülle aus einem Emulgator, wie beispielsweise einem Phospholipid, enthält, wobei die Hülle einen Liganden für eine zielgerichtete Bindung enthält.

5. Verwendung nach Anspruch 3 oder 4, wobei die Emulsion Nanopartikel mit einem Kern umfasst, der das Perfluor-tert-Butylcyclohexan umgeben von einer Hülle aus einem Emulgator, wie beispielsweise einem Phospholipid, enthält, wobei die Hülle ein Kontrastmittel für die Bildgebung enthält, das aus der Gruppe bestehend aus paramagnetischen Kontrastmitteln, radioaktiven Markern und Gemischen aus diesen ausgewählt wird.

6. Kontrastmittel zur Verwendung in einem an einem menschlichen oder einem Tierkörper durchgeführten diagnostischen Bildgebungsverfahren, das 19F-MRT umfasst, wobei das Kontrastmittel eine wässrige Emulsion aus Nanopartikeln mit einem Kern umfasst, der das Perfluor-tert-Butylcyclohexan umgeben von einer Hülle aus einem Emulgator, wie beispielsweise einem Phospholipid, enthält, wobei die Hülle zumindest eine weitere Bildgebungsfunktion umfasst, die aus der Gruppe bestehend aus Liganden für eine zielgerichtete Bindung, 1H-MRT-Kontrastmitteln, radioaktiven Markern und Gemischen aus diesen ausgewählt wird.

7. Perfluor-tert-Butylcyclohexan zur Verwendung als Kontrastmittel für die molekulare 19F-Resonanzbildgebung in einem an einem menschlichen oder einem Tierkörper durchgeführten diagnostischen Bildgebungsverfahren.

8. Perfluor-tert-Butylcyclohexan zur Verwendung in einem an einem menschlichen oder einem Tierkörper durchgeführten diagnostischen Verfahren nach Anspruch 7, wobei das Kontrastmittel eine wässrige Emulsion des Perfluor-tert-Butylcyclohexan umfasst.

9. Perfluor-tert-Butylcyclohexan zur Verwendung in einem an einem menschlichen oder einem Tierkörper durchgeführten diagnostischen Verfahren nach Anspruch 8, wobei die Emulsion Nanopartikel mit einem Kern umfasst, der das Perfluor-tert-Butylcyclohexan umgeben von einer Hülle aus einem Emulgator, wie beispielsweise einem Phospholipid, enthält, wobei die Hülle einen Liganden für eine zielgerichtete Bindung enthält.

10. Perfluor-tert-Butylcyclohexan zur Verwendung in einem an einem menschlichen oder einem Tierkörper durchgeführten diagnostischen Verfahren nach Anspruch 8 oder 9, wobei die Emulsion Nanopartikel mit einem Kern umfasst, der das Perfluor-tert-Butylcyclohexan umgeben von einer Hülle aus einem Emulgator, wie beispielsweise einem Phospholipid, enthält, wobei die Hülle ein Kontrastmittel für die Bildgebung enthält, das aus der Gruppe bestehend aus paramagnetischen Kontrastmitteln, radioaktiven Markern und Gemischen aus diesen ausgewählt wird.

11. Perfluor-tert-Butylcyclohexan zur Verwendung in einem an einem menschlichen oder einem Tierkörper durchgeführten diagnostischen Verfahren nach einem der Ansprüche 7 bis 10, wobei das Kontrastmittel intravenös injiziert oder in den Gastrointestinaltrakt eingeleitet wird.

12. Perfluor-tert-Butylcyclohexan zur Verwendung in einem an einem menschlichen oder einem Tierkörper durchgeführten diagnostischen Verfahren nach einem der Ansprüche 7 bis 10, wobei zusätzlich zu dem Kontrastmittel Perfluor-tert-Butylcyclohexan ein zweites 19F-MRT-Kontrastmittel, vorzugsweise Perfluoroctylbromid, verabreicht wird.

13. Perfluor-tert-Butylcyclohexan zur Verwendung in einem an einem menschlichen oder einem Tierkörper durchgeführten diagnostischen Verfahren nach Anspruch 12, wobei das Kontrastmittel Perfluor-tert-Butylcyclohexan mit einem oder mehreren Liganden für ein erstes Target, wie beispielsweise einem ersten Onkologiemarker, versehen ist und das zweite 19F-MRT-Kontrastmittel mit einem oder mehreren Liganden für ein zweites Target, wie beispielsweise einem zweiten, von dem ersten verschiedenen Onkologiemarker, versehen ist.

## Revendications

1. Utilisation du perfluoro-t-butyl cyclohexane comme agent de contraste en imagerie, de préférence l'imagerie moléculaire.

2. Utilisation selon la revendication 1, dans laquelle l'imagerie comprend l'imagerie par résonance magnétique du ¹⁹F.

3. Utilisation selon la revendication 1 ou 2, dans laquelle l'agent de contraste comprend une émulsion aqueuse du perfluoro-tert-butyl cyclohexane.

4. Utilisation selon la revendication 3, dans laquelle l'émulsion comprend des nanoparticules possédant un noyau comprenant le perfluoro-tert-butyl cyclohexane entouré d'une coque composée d'un agent émulsifiant, tel qu'un phospholipide, la coque comprenant un ligand permettant une liaison ciblée.

5. Utilisation selon la revendication 3 ou 4, dans laquelle l'émulsion comprend des nanoparticules possédant un noyau comprenant du perfluoro-tert-butyl cyclohexane entouré d'une coque composée d'un agent émulsifiant, tel qu'un phospholipide, la coque comprenant un agent de contraste d'imagerie choisi dans le groupe constitué des agents paramagnétiques, des marqueurs radioactifs et de leurs mélanges.

6. Agent de contraste destiné à être utilisé dans un procédé d'imagerie diagnostique réalisé sur le corps d'un humain ou d'un animal comprenant une IRM du ¹⁹F, l'agent comprenant une émulsion aqueuse de nanoparticules possédant un noyau comprenant du perfluoro-tert-butyl cyclohexane entouré d'une coque composée d'un agent émulsifiant, tel qu'un phospholipide, la coque comprenant au moins une autre fonctionnalité d'imagerie, choisi dans le groupe constitué d'un ligand permettant une liaison ciblée, des agents de contraste pour l'IRM du ¹H, des marqueurs radioactifs et de leurs mélanges.

7. Perfluoro-tert-butyl cyclohexane, destiné à être utilisé comme agent de contraste en imagerie par résonance moléculaire du ¹⁹F dans un procédé d'imagerie diagnostique réalisé sur le corps d'un humain ou d'un animal.

8. Perfluoro-tert-butyl cyclohexane destiné à être utilisé dans un procédé de diagnostic réalisé sur le corps d'un humain ou d'un animal selon la revendication 7, dans lequel l'agent de contraste comprend une émulsion aqueuse du perfluoro-tert-butyl cyclohexane.

9. Perfluoro-tert-butyl cyclohexane destiné à être utilisé dans un procédé de diagnostic réalisé sur le corps d'un humain ou d'un animal selon la revendication 8, dans lequel l'émulsion comprend des nanoparticules possédant un noyau comprenant le perfluoro-tert-butyl cyclohexane entouré d'une coque composée d'un agent émulsifiant, tel qu'un phospholipide, la coque comprenant un ligand permettant une liaison ciblée.

10. Perfluoro-tert-butyl cyclohexane destiné à être utilisé dans un procédé de diagnostic réalisé sur le corps d'un humain ou d'un animal selon la revendication 8 ou 9, dans lequel l'émulsion comprend des nanoparticules possédant un noyau comprenant le perfluoro-tert-butyl cyclohexane entouré d'une coque composée d'un agent émulsifiant, tel qu'un phospholipide, la coque comprenant un agent de contraste d'imagerie choisi dans le groupe constitué des agents paramagnétiques, des marqueurs radioactifs et de leurs mélanges.

11. Perfluoro-tert-butyl cyclohexane destiné à être utilisé dans un procédé de diagnostic réalisé sur le corps d'un humain ou d'un animal selon l'une quelconque des revendications 7 à 10, dans lequel l'agent de contraste est injecté par voie intraveineuse ou est introduit dans le tractus gastro-intestinal.

12. Perfluoro-tert-butyl cyclohexane destiné à être utilisé dans un procédé de diagnostic réalisé sur le corps d'un humain ou d'un animal selon l'une quelconque des revendications 7 à 11, dans lequel un second agent de contraste pour IRM du ¹⁹F, de préférence le bromure d'octyle perfluoré, est utilisé en plus de l'agent de contraste perfluoro-tert-butyl cyclohexane.

13. Perfluoro-tert-butyl cyclohexane destiné à être utilisé dans un procédé de diagnostic réalisé sur le corps d'un humain ou d'un animal selon la revendication 12, dans lequel l'agent de contraste perfluoro-tert-butyl cyclohexane est pourvu d'un ou de plusieurs ligands pour une première cible, comme un premier marqueur oncologique, et le second agent de contraste pour IRM du ¹⁹F est pourvu d'un ou de plusieurs ligands pour une seconde cible, comme un second marqueur oncologique différent du premier.
